# EUROPÄISCHE PATENTSCHRIFT

(11) **EP 1 311 851 B1**
(45) Veröffentlichungstag und Bekanntmachung des Hinweises auf die Patenterteilung: **25.10.2006**
(21) Anmeldenummer: 01965053.0
(22) Anmeldetag: 30.06.2001
(51) Int. Cl.: G01N 33/50, C12Q 1/02

(54) **SCREENINGVERFAHREN ZUR FINDUNG VON DIE ENDOCYTOSE FÖRDERNDEN HILFSSTOFFEN**
SCREENING METHOD FOR DISCOVERING AUXILIARY AGENTS PROMOTING ENDOCYTOSIS
PROCEDE DE CRIBLAGE DESTINE A DECOUVRIR DES AGENTS AUXILIAIRES FAVORISANT L'ENDOCYTOSE

(30) Priorität: 23.08.2000 DE 10041411
(43) Veröffentlichungstag der Anmeldung: 21.05.2003
(73) Patentinhaber: RiNA Netzwerk RNA-Technologie GmbH, 14195 Berlin (DE)
(72) Erfinder: LANG, Christine, 10625 Berlin (DE); GESSNER, Reinhard, 14193 Berlin (DE); NEUKAMM, Birgit, 10961 Berlin (DE); PRINZ, Bianka, 10587 Berlin (DE); NEVOIGT, Elke, 10439 Berlin (DE)
(74) Vertreter: Jungblut, Bernhard Jakob
(86) Internationale Anmeldenummer: PCT/EP2001/007509
(87) Internationale Veröffentlichungsnummer: WO 2002/016935

(56) Entgegenhaltungen:
- WO-A-98/06869
- WO-A-99/13719
- HUGHES JA ET AL: "Evaluation of adjuvants that enhance the effectiveness of antisense oligodeoxynucleotides" PHARMACEUTICAL RESEARCH, NEW YORK, NY, US, Bd. 13, Nr. 3, März 1996 (1996-03), Seiten 404-410, XP002100198 ISSN: 0724-8741
- PLANK C ET AL: "THE INFLUENCE OF ENDOSOME-DISRUPTIVE PEPTIDES ON GENE TRANSFER USING SYNTHETIC VIRUS-LIKE GENE TRANSFER SYSTEMS" JOURNAL OF BIOLOGICAL CHEMISTRY, AMERICAN SOCIETY OF BIOLOGICAL CHEMISTS, BALTIMORE, MD, US, Bd. 269, Nr. 17, 29. April 1994 (1994-04-29), Seiten 12918-12924, XP000615488 ISSN: 0021-9258
- ERBACHER PATRICK ET AL: "Putative role of chloroquine in gene transfer into a human hepatoma cell line by DNA/lactosylated polylysine complexes." EXPERIMENTAL CELL RESEARCH, Bd. 225, Nr. 1, 1996, Seiten 186-194, XP002188020 ISSN: 0014-4827
- CRISTIANO RICHARD J: "Targeted, non-viral gene delivery for cancer gene therapy." FRONTIERS IN BIOSCIENCE, Bd. 3, Nr. CITED DEC. 1, 1998, 15. November 1998 (1998-11-15), Seiten D1161-1170, XP001053037
- TERNG HARN-JING ET AL: "Human transferrin receptor is active and plasma membrane-targeted in yeast." FEMS MICROBIOLOGY LETTERS, Bd. 160, Nr. 1, 1. März 1998 (1998-03-01), Seiten 61-67, XP001053018 ISSN: 0378-1097

## Beschreibung

### Gebiet der Erfindung

Die Erfindung betrifft ein Screeningverfahren zur Findung von die Endocytose von nieder- und/oder makromolekularen Verbindungen, insbesondere Nukleinsäuren, in Zellen fördernden Hilfsstoffen sowie die Verwendung von mittels eines solchen Screeningverfahrens gefundenen Hilfsstoffen zur Herstellung von pharmazeutischen Zusammensetzungen.

Der Begriff der Endocytose bezeichnet die Aufnahme von extracellulärem, korpuskulärem oder gelöstem, meist makromolekularem Material in eine Zelle. Makromolekular meint Verbindungen mit einem Molekulargewicht oberhalb von 300 da, vorzugsweise von oberhalb 500 da, höchstvorzugsweise von oberhalb 1000 da. Zu den natürlicherweise aufnehmbaren Makromolekülen gehören beispielsweise Antigen-Antikörper-Komplexe, Lipoproteine, LDL, Transferrin und Nukleinsäuren. Natürlicherweise aufnehmbare korpuskuläres Material umfaßt beispielsweise Viren, Bakterien und Protozoen.

Unter anderem im Rahmen gentherapeutischer Maßnahmen, aber auch bei der Nutzung von direkt translatierbaren RNA-Molekülen oder als Antisense-RNA, Ribozyme oder RNA-Aptamere wirkenden Nukleinsäuren (einschließlich nicht-natürliche Derivate wie PNA) müssen die makromolekularen Nukleinsäuren zumindest als ersten Schritt in das Cytosol aufgenommen, ggf. auch weiter in den Kern transportiert werden. Methoden und Hilfsstoffe, wie bei den im Rahmen der pharmazeutischen Industrie bislang favorisierten niedermolekularen Wirkstoffe sind hierfür ungeeignet. Vielmehr müssen die natürlichen Endocytosemechanismen genutzt und die Transportrate von der Zellmembran bis zum Wirkort durch geeignete Hilfsmittel auf brauchbare Levels angehoben werden.

Hilfsstoffe sind solche Substanzen, die auf einen oder mehrere Schritte der Endocytose die Transportrate erhöhend einwirken bzw. diese modulieren.

### Hintergrund der Erfindung und Stand der Technik

Die Gentherapie ist eine vielversprechende Methode zur Heilung von einer Vielzahl von Krankheiten, die durch einen keimbahngängigen oder somatischen Gendefekt hervorgerufen werden, wie Erbkrankheiten und Krebs (Garret MC, Crit Rev Ther Drug Carrier Syst 1999; 16(2): 147-207). Bei der Gentherapie werden Nukleinsäuren in die Zielzelle eingebracht, die dort den Defekt direkt beheben sollen.
In den letzten Jahrzehnten wurden mehrere Verfahren zur Einbringung von Nukleinsäuren in die Zelle entwickelt. Dazu zählt das Einbringen von Nukleinsäuren in die Zelle mittels viraler Vektoren, die Lipofektion und die gerichtete Aufnahme mittels rezeptorvermittelter Endocytose, wie z. B. die Transferrinfektion. Die Nachteile viraler Systeme gegenüber den restlichen Systemen wurden in den letzten Jahren immer deutlicher, so daß die Zukunft der Gentherapie wahrscheinlich in der Benutzung der anderen Systeme liegt, z.B. der Transferrinfektion. Dieses ist ein Verfahren, das sich der natürlichen Endocytosemechanismen der Zelle bedient. Die Transferrinfektion beruht auf dem natürlichen Transferrin-Transferrinrezeptor-Endocytosesystem, das die Eisenversorgung in proliferierenden, differenzierenden und Hämoglobin-synthetisierenden, humanen Zellen gewährleistet (Theil, E.C., Aisen, P. (1987) The storage and transport of iron in animal cells. Iron transport in Microbes, Plants, Animals, pp. 491-520, Winkelmann, G., van der Helm, D. und Neilands, J.B.( Herausgeber), VCH, Weinheim). Beim Transferrinfektionssystem wurde das natürlich vorkommenden Transferrin-Transferrinrezeptor-Endocytosesystems modifiziert, indem die DNA an den Liganden Transferrin gekoppelt wurde (E. Wagner et al., 1991, Bioconjugate Chem. 2:226-231). Der Ligand bindet spezifisch an den Zelloberflächenrezeptor der Zielzelle und wird durch Endocytose aufgenommen. Um die negative Ladung der DNA zu neutralisieren, die DNA zu kondensieren und sie somit einer Aufnahme zugänglich zu machen, werden Polykationen wie Polyethylenimin oder Polylysin eingesetzt. Es konnte außerdem gezeigt werden, daß auch kondensierte DNA, die an keinen Liganden gekoppelt ist, endocytotisch aufgenommen wird (W.T. Godbey et al., 1996, PNAS 96:5177-5181) und dieses System wird als Polyfektion beschrieben. Für eine gentherapeutische Nutzung der bisher entwickelten Systeme zum zielgerichteten Einbringen von Nukleinsäuren in eukaryontische Zellen durch Endocytose ist jedoch die Aufnahmerate, Stabilisierung der DNA vor Degradation in den zelleigenen Kompartimenten und Transfereffizienz an den Wirkungsort (Cytosol oder Kern) unzureichend (Mahato RI (1999) Non-viral peptide-based approaches to gene delivery. J Drug Target. 7(4):249-68).

Neben der Gentherapie gibt es weitere Behandlungsansätze und Nutzung meist makromolekularer Nukleinsäuren. Ein erster Ansatz besteht in der Einschleusung von direkt translatierbarer RNA in eine Zielzelle, wobei dann ein durch die RNA codierter Wirkstoff durch die zelleigenen Mechanismen exprimiert wird. Mittels Antisense RNA, RNA-Aptameren und Ribozymen können in einer Zelle aufgrund von Fehlfunktionen, Mutationen, etc. natürlicherweise ablaufenden Prozesse auf RNA-Ebene moduliert werden. Auch in diesem Zusammenhängen ist die Nutzung und Modulation bzw. Förderung natürlicher Endocytoseprozesse erforderlich, damit die Nukleinsäure in ausreichend großer Menge in die Zelle eingeschleust wird.

Entsprechendes gilt für andere nieder- und/oder makromolekulare Moleküle in therapeutischen Zusammenhängen, die nur schwer oder gar nicht die Membranbarriere überwinden können und im Komplex mit Proteinen, Nukleinsäuren oder synthetischen Makromolekülen mittels Endocytose in die Zelle aufgenommen werden können. Hierzu gehören insbesondere alle Medikamente, die im Plasma in ionisierter Form vorliegen und für die kein geeignetes zelluläres Transportsystem existiert. Weiterhin gilt. Weiterhin ist es wünschenswert, ein Verfahren zur Verfügung zu haben für die Aufnahme von lipophilen Pharmaka, die eine hohe Eiweissbindung aufweisen und nach in vitro Bindung an ein geeignetes Carrier-Protein oder ein anderes geeignetes Makromolekül über Endocytose in die Zielzelle aufgenommen werden können.

### Technisches Problem der Erfindung

Der Erfindung liegt das technische Problem zugrunde, die Transportrate aus den endosomalen Vesikeln an den Zielort, die Aufnahmerate, die Stabilisierung und die Transfereffizient nieder- und/oder makromolekularer Verbindungen, insbesondere von Nukleinsäuren, in die natürliche Endocytosemechanismen nutzenden Verfahren zur Einbringung von makromolekularen Verbindungen, insbesondere Nukleinsäuren, in Zellen zu verbessern. Der Erfindung liegt das weitere Problem zugrunde, eine Verfahren zur Findung von Hilfsstoffen anzugeben, mittels welcher die vorstehenden Verbesserungen erreicht werden können.

### Grundzüge der Erfindung

Zur Lösung dieses technischen Problems lehrt die Erfindung ein Screeningverfahren zur Findung von die Endocytose von nieder- und/oder makromolekularen Verbindungen, insbesondere Nukleinsäuren, in Zellen fördernden Hilfsstoffen mit den folgenden Verfahrensschritten: a) es wird ein Hefestamm ausgewählt und hieraus werden Hefezellen kultiviert, b) die Hefezellen werden mit einem potentiellen, die Endocytose von nieder- und/oder makromolekularen Verbindungen, insbesondere Nukleinsäuren, in Zellen fördernden Hilfsstoff sowie mit einer replizierbaren Nukleinsäure und/oder im Kern und/oder im Cytosol nachweisbaren nieder-und/oder makromolekulare Verbindung, insbesondere Nukleinsäure, inkubiert, c) es wird die Transformationsrate oder die Transportrate in das Cytosol oder den Kern bestimmt und auf Erhöhung der Transformationsrate oder der Transportrate, verglichen mit der Transformationsrate oder der Transportrate in einem Referenzversuch ohne Einsatz des Hilfsstoffes, untersucht, d) der Hilfsstoff wird selektiert oder verworfen nach Maßgabe des erhaltenen Wertes der Erhöhung der Transformationsrate in Stufe c). Der Ausdruck der Endocytose umfaßt in Rahmen der Erfindung auch den Transport von Verbindungen an ihren Wirkungsort in einer Zielzelle. Nukleinsäuren können an Liganden gebunden bzw. gekoppelt sein. Der Ligand wird im unmodifierten und modifizierten Zustand durch rezeptorvermittelte Endocytose aufgenommen und führt zur stabilen Transformation der Hefezelle (siehe z.B. Neves C., Escriou V. Byk G., Scherman D., Wils P. Cell Biol. Toxicol. (1999); 15(3):193-202 oder Neves C., Byk G., Scherman D., Wils P, FEBS Letters (1999) 453:41-45). Es versteht sich, daß im Rahmen der Erfindung auch mit mehreren Hilfstoffen zugleich, sei es nieder-oder makromolekular oder beides in Mischung, gearbeitet werden kann.

Der Referenzversuch wird vorzugsweise durchgeführt durch Ersatz der Verfahrenstufe b) durch die folgende Verfahrenstufe: b') die Hefezellen werden mit einer replizierbaren Nukleinsäure und/oder im Kern und/oder im Cytosol der Hefezellen nachweisbaren makromolekularen Verbindung, insbesondere Nukleinsäure, inkubiert, bei ansonsten unveränderten anderen Verfahrenstufen.

In Hinblick auf eine Testung selektierter Hilfstoffe zur Verwendung für humane Zellen empfiehlt es sich, daß die folgende Verfahrenstufe angeschlossen wird: e) ein in Stufe d) selektierter potentieller Hilfsstoff wird einer Bestimmung der Erhöhung der Transformationsrate oder der Transportrate gegenüber einer Transformationsrate oder Transportrate ohne Einsatz des Hilfsstoffes bei Vertebratenzellen unterworfen und der Hilfsstoff wird selektiert oder verworfen nach Maßgabe der Erhöhung der Transformationsrate oder Transportrate.

Besonders bevorzugt ist eine Ausführungsform der Erfindung, wobei parallel oder nacheinander die Erhöhung der Transformationsrate oder der Transportrate für ein und denselben potentiellen Hilfsstoff mit wildtyp sowie mutierten Hefezellen durchgeführt wird.

In einer Variante der Erfindung wird ein Screeningverfahren zur Findung von die Endocytose von Nukleinsäuren in Zellen fördernden Hilfstoffen mit den folgenden Verfahrensschritten gelehrt: a) es wird ein Vertebratenzellenstamm ausgewählt und hieraus werden Zellen kultiviert, b) die Zellen werden mit einem potentiellen, die Endocytose von Nukleinsäuren in Zellen fördernden Hilfsstoff sowie mit einer in den Vertebratenzellen transkribierbaren DNA und/oder mit einer direkt oder indirekt nachweisbaren RNA inkubiert, c) die Zellen der Stufe b) werden untersucht auf die Gesamtmenge aufgenommener und adsorbierter Nukleinsäure, die Menge an intrazellulärer Nukleinsäure, die Menge an kernständiger Nukleinsäure, die Transfektionsrate und/oder die Menge an Genprodukt, verglichen mit einem Referenzversuch ohne Einsatz des Hilfsstoffes, d) der Hilfsstoff wird selektiert oder verworfen nach Maßgabe der Ergebnisse aus Stufe c)

Schließlich lehrt die Erfindung die Verwendung eines Hilfsstoffs, erhältlich durch ein erfindungsgemäßes Verfahren, zur Herstellung einer pharmazeutischen Zusammensetzung zur Förderung der Aufnahme von Makromolekularen Verbindungen, insbesondere Nukleinsäuren, in Zellen.

Der Erfindung liegt die Erkenntnis zugrunde, daß für das Screening von Substanzen, die zu einer Steigerung der endocytotischen Aufnahme von makromolekularen Verbindungen, insbesondere Nukleinsäuren, in die Zielzelle sowie deren Translokation an den Wirkungsort führen, Hefe, insbesondere der Organismus Saccharomyces cerevisiae, ein besonders gut geeignetes Modellsystem bildet. Die grundlegenden Mechanismen der Aufnahme und des Transportes scheinen zwischen verschiedenen eukaryontischen Organismen nämlich weitgehend konserviert zu sein. Die intrazellulären Transportvorgänge bei Hefe sind sehr gut untersucht. Für die Hefe Saccharomyces cerevisiae wurde beobachtet, daß in der Plasmamembran lokalisierte Rezeptoren, wie der alpha-Faktor-Rezeptor, endocytiert und über die Endosomen zur Vakoule transportiert werden, wo sie abgebaut werden (Schandel, K.Jenness, D. (1994) Direct evidence for ligand-induced internalization of the yeast alpha-faktor pheromone receptor. Mol Cell. Biol., 14:7245-7255; Riballo, E., Herweijer, M., Wolf, D., Lagunas, R. (1995) Catabolit inactivation of the yeast maltose transporter occurs in the vacuole after internalization by endocytosis. J. Bacteriology, 177: 5622-5627). Ein "Recycling", bei welchem der Rezeptor nach der Aufnahme in die Zelle über Endocytose aus den Endosomen zurück an die Plasmamembran transportiert wird, ist in Hefe im Gegensatz zu Säugertierzellen noch nicht beschrieben worden. Säugerzellen bringen z. B. den Transferrinrezeptor/Transferrin-Komplex über ein "Recycling" aus den Endosomen zurück in die Plasmamembran. In Säugerzellen gibt es jedoch deutliche Hinweise, daß modifiziertes Transferrin sowie Transferrin-DNA-Komplexe nicht "recycelt" werden, sondern in den Lysosomen degradiert werden (Wagner, E. , Curiel, D., Cotton, M.(1994) Delivery of drugs, proteins and genes into cells using transferrin as a ligand for receptor-induced Endocytosis, Advanced Drug Delivery Reviews, 14: 113-135) Daher eignet sich die Hefe, insbesondere Saccharomyces cerevisiae, sehr gut als Modell für die Aufnahme von nieder- und/oder Makromolekularen Verbindungen, beispielsweise Nukleinsäuren bzw. Nukleotiden, in die humane Zelle durch Endocytose. Es ist also davon auszugehen, daß jedenfalls ein großer Teil der mit der Erfindung gefundenen hocheffektiven Hilfsstoffe auch im humanen System eine hohe Aufnahmerate, eine gute Stabilisierung und eine hohe Transfereffizienz zeigen.

Die Erfindung beruht auf der weiteren Erkenntnis, daß bei Hefezellen sich im Gegensatz zu Säugetierzellen in kurzer Zeit eine Vielzahl von Defektmutanten herstellen lassen, bei denen definierte Gene des Endocytoseweges gezielt unterbrochen werden. Der Einsatz der Mutanten macht es möglich, die einzelnen Schritte des Endocytoseweges von der Plasmamembran durch die endocytotischen Vesikeln bis zur Vakuole experimentell zu trennen und ihren Einfluß auf den Transfer ins Cytosol bzw. den Kern der Zelle zu bestimmen. Basierend aus den daraus gewonnenen Erkenntnissen können Substanzen dahingehend getestet werden, ob sie die gefundenen Engpässe bei der Aufnahme und Translokation aus den endocytotischen Vesikeln an den Zielort durchbrechen können. Mit anderen Worten ausgedrückt, die Erfindung kann auch zur Erforschung der Funktionsmechanismen einzelner Hilfsstoffe genutzt werden. Da der Prozess der Aufnahme makromolekularere Verbindungen bzw. Nukleinsäureaufnahme in einer Zelle vielstufig ist, können auf diese Weise auch Hilfsstoffkombinationen ermittelt werden, welche insgesamt den erstrebten Effekt erzielen. Mit solchen Hilfsstoffkombinationen können Aufnahmerate und Transfereffizienz potenziert werden, da alle Engpässe gezielt und spezifisch beeinflußt werden.

Die Hefezellen haben gegenüber Säugertierzellen außerdem den Vorteil einer sehr viel einfacheren Handhabung. Sie können einfach und in großen Zellmengen kultiviert werden und eine große Anzahl von Mutanten oder Transformanten können parallel analysiert werden. Wegen der oben genannten Eigenschaften eignen Hefen sich gut für Versuchsanordnungen, die wegen der großen Anzahl an Parallelansätzen eine Miniaturisierung erforderlich machen. Gegenüber humanen Systemen wird also im Ergebnis ein sehr hoher Durchsatz beim Screenen erreicht.

Die Transportrate kann durch eine zeitabhängige, PCR-gestützte Messung der Menge an Nukleinsäure in den verschiedenen zellulären Kompartimenten (ganze Zellen, DNase verdaute ganze Zellen, Zellkerne) sowie ihrer biologischen Aktivität (GFP, Luciferase-Messung) bestimmt werden. Bei genauer Kenntnis der absoluten Mengen kann die Transportrate exakt berechnet werden. Für die Messung des Einflusses eines Hilfsstoffes auf einzelne Schritte des Transports ist jedoch in der Regel ein einfacher Vergleich der Menge an Nukleinsäuren in den verschiedenen Kompartimenten in Anwesenheit und Abwesenheit des Hilfsstoffes hinreichend.

Die Transportrate läßt sich auch über die Transformationsrate, gemessen gemäß dem Beispiel 1, definieren. Entsprechende vergleichende Messungen bezüglich verschiedener intrazellulärer Ziel-Kompartimente lassen sich unschwer entwickeln. Stets ist dabei gegenüber einem Referenzversuch ohne Hilfsstoff unter ansonsten identischen Versuchsbedingungen zu vergleichen. Alternativ kann gegenüber dem Einsatz einer definierten Menge bzw. Konzentration an Chloroquin als Standard verglichen werden.

Im Rahmen der Erfindung ist es auch möglich, den Einfluß von sonstigen Bedingungen, wie Temperatur, Druck, elektrische Felder, elektromagnetische Wechselfelder, Strom und Eigenschaften sowie Zusammensetzung des Mediums zu untersuchen.

### Ausführungsformen der Erfindung

In Hinblick auf eine effiziente und mit hoher Durchsatzkapazität arbeitenden Untersuchung wird es bevorzugt, wenn eine Mehrzahl von unterschiedlichen potentiellen, die Endocytose von makromolekularen Verbindungen, insbesondere Nukleinsäuren, in Zellen fördernde Hilfstoffe parallel auf Erhöhung der Transformationsrate oder der Transportrate untersucht wird. Dies läßt sich besonders vorteilhaft mit Hefezellen durchführen, da sie wesentlich einfacher handhabbar sind als Säugertierzellen und einfach und in großen Zellmengen kultiviert werden können. Zudem lassen sich unschwer eine Vielzahl von Mutanten mit untersuchen, wodurch schnell Informationen auch über die Wirkorte der Hilfsstoffe im Endocytoseprozeß erhalten werden. Schließlich erlaubt es die einfache Handhabbarkeit, auch eventuelle Hilfsstoffkombinationen effektiv und mit vergleichsweise wenig Zeitaufwand zu untersuchen.

Bevorzugterweise ist der Hefezellenstamm ausgewählt aus der Gruppe bestehend aus "RPY10, 4STLU, AH22, DBY747, GRF18, CBS6503, ABYS86, MF9, YMTA, BJ3505, GAG2", insbesondere RPY10 ist. Bei Einsatz von RPY10 ist es bevorzugt, wenn mutierte Hefezellenstämme ausgewählt sind aus der Gruppe bestehend aus "Deletionsmutanten des Stamms RPY10 mit Deletion der Gene YPT51, YPT7, VPS27 und/oder PEP4". Generell können Mutantenstämme eingesetzt werden, die den Transport an endocytierten Material an definierten Stellen des Endocytoseweges unterbrechen, bzw. denen spezifische DNA/RNA degradierende Enzyme fehlen. Diese Deletionsmutanten werden aus den Ausgangsstämmen konstruiert, so daß sie sich nur in der Deletion des für den endocytotischen Transport relevanten Gens unterscheiden (siehe z.B. Güldener U., Heck S., Fiedler T., Beinhauer J. , Hegemann JH, A new efficient gene disruption casette for repeated use in budding yeast. Nucleic Acids Research, 1996, 24 (13): 2519-2524) Möglicherweise können auch temperatursensitive Stämme verwendet werden, die bei einer Temperatur oberhalb der optimalen Wachstumstemperatur einen Defekt ausbilden.

Grundsätzlich können in der Variante unter Einsatz von Vertebratenzellen beliebige Zellinien, primäre Zellen oder Gewebeexplantate eingesetzt werden. Bevorzugterweise werden Säugetierzellinien, idealerweise humane Zellinien verwendet. Zweckmäßig ist es, wenn die Vertebratenzellinie ausgewählt ist aus der Gruppe bestehend aus "HepG2, K562, B16, MeWo, CMT-93, RMA, LL/2, 293, HT-1080, COS-1, COS-2, CV-1, CHO, OCM, HL-60, L929, 3T3, BLK CL.4, HeLa, T84", insbesondere HepG2 ist.

In einer Ausführungsform der Erfindung mit Bestimmung der Transfektionsrate und/oder der Menge an Genprodukt kann die Nukleinsäure ein Markergen enthalten, vorzugsweise das Luciferasegen und/oder EGFP, und wobei die Erhöhung der Transfektionsrate durch Auszählen der das Markergen exprimierenden Zellen oder die Luziferase Aktivität bestimmt wird. Bei der Bestimmung der Transportraten von makromolekularen Verbindungen, auch bei Nukleinsäuren, kann allgemein auch mit anderen Methoden gearbeitet werden, beispielsweise mit radioaktiven Markern, Fluorophoren oder chemisch modizierten Verbindungen, welche einen sekundären qualitativen und/oder quantitativen Nachweis ermöglichen. Im Rahmen der Nukleinsäuren kommen auch RNA-Moleküle, welche direkt translatierbar sind und für ein nachweisbares Protein kodieren, oder RNA-Moleküle, die eine anderweitig nachweisbare Funktion in der Zelle ausüben, wie z.B. Antisense-RNA, Ribozyme oder RNA-Aptamere in Frage.

Auswertungstechnisch ist es bevorzugt, wenn die Transformationsrate oder Transportrate auf die Anzahl teilungsfähiger Zellen aus der Stufe b) bezogen wird durch Bestimmung der Anzahl der kolonienbildenden Einheiten nach Kultivierung der Zellen aus Stufe b) in auf die Nukleinsäure abgestimmtem Selektionsmedium und Division durch die Anzahl der kolonienbildenden Einheiten nach Kultivierung der Zellen aus Stufe b) in Vollmedium. Eine erfolgreiche Aufnahme von beispielsweise doppelsträngiger, circulärer DNA und führt zur Expression eines Markergens, für das der Hefestamm auxotroph ist. Eine Quantifizierung erfolgt über die Anzahl der kolonienbildenden Einheiten auf Selektionsmedium, welche auf die Anzahl teilungsfähiger Zellen normiert wird, um eine eventuelle Toxizität eines der zu testenden Hilfsstoffe auf die Zellen zu berücksichtigen.

Die Verifizierung der erfolgreich transformierten bzw. transfizierten Zellen, sowie die Bestimmung der Verteilung der Nukleinsäuren in verschiedenen intrazellulären Kompartimenten, und damit die jeweilige Transformationsrate bzw. Transportrate könen mittels PCR durchgeführt werden.

Im folgenden wird die Erfindung anhand von Ausführungsbeispielen näher erläutert.

### Beispiel 1:

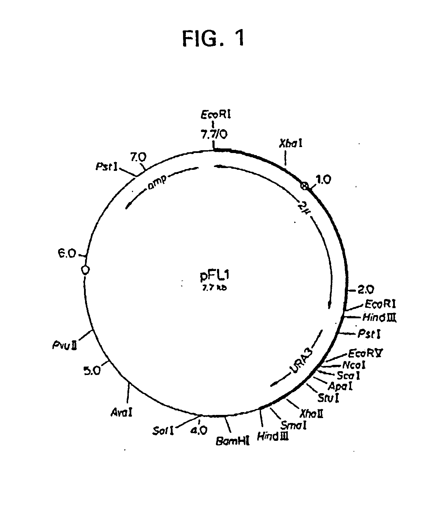

Ein Testverfahren zur Quantifizierung der Aufnahme und des endocytotischen Transfers wurde wie folgt durchgeführt. 1*10⁹ Zellen aus der logarithmischen Wachstumsphase des Stammes RPY10 (Matα, leu2-3 ura3, his4 ade6) (4*10⁷-7*10⁷ Zellen pro ml)(Piper, R.C., Whitters, E.A., Stevens T.H. (1994) Yeast VpsS4p is a Sec lp-like protein required for the consumption of vacuolar targeted, post-Golgi vesicels. Eur. J. Cell Biol. 65:305-318) wurden mit 10 µg des gereinigten Plasmids YEP24 (siehe: D. Botstein et al., 1979, Gene 8:17-24) 24h inkubiert und zwar einmal mit und einmal ohne Hilfsstoff. Der Aufbau des Plasmids ist in der Figur 1 dargestellt.

Die Zellen wurden vor der Inkubation mit dem Plasmid mit mindestens 2x dem gleichem Volumen H₂O gewaschen und in 1ml 1M Saccharose (pH 3,3, hohe Konzentrationen fermentierbarer Zucker) aufgenommen, mit der Pipettenspitze resuspendiert und in je ein 10 ml Falkonröhrchen überführt. Sofort nach diesem Schritt wurden in 1 Röhrchen 5 µl Chloroquin als Hilfsstoff (Stammlsg. 100 mM) dazupipettiert und in ein zweites nicht. Beide wurden 5 min bei Raumtemperatur inkubiert.

Danach wurden 10 µg DNA aus derselben DNA-Aufarbeitung dazupipettiert (in 3-100 µl). Die Falkon-Röhrchen wurden fest zugeschraubt und 24 Stunden in Wasserbad oder Brutraum schütteln gelassen bei 24°C (die optimale Transformationsrate liegt bei 24°C-28°C). Aus praktischen Gründen kann auch über Nacht inkubiert werden.

Nach der Inkubation wurden 200-500 Zellen auf YE-Platten (Vollmedium) pro Platte ausplattiert und die koloniebildenden Einheiten (KBE)bestimmt. Die restlichen Zellen wurden auf 3-5 Platten mit Selektionsmedium ausplattiert. Nach 5 Tagen bei 24°C wurden die KBE ausgezählt und mittels PCR geprüft.

Zur Auswertung wurden die Transformanten pro eingesetzten Zellen auf die Anzahl teilungsfähiger Zellen nach der Inkubation bezogen. Es ergaben sich Werte für die Transformationsrate von 10,2 [10⁻⁸ Transformanten/ KBE] mit Chloroquin und von 0,8 [10⁻⁸ Transformanten/KBE] ohne Chloroquin.

### Beispiel 2:

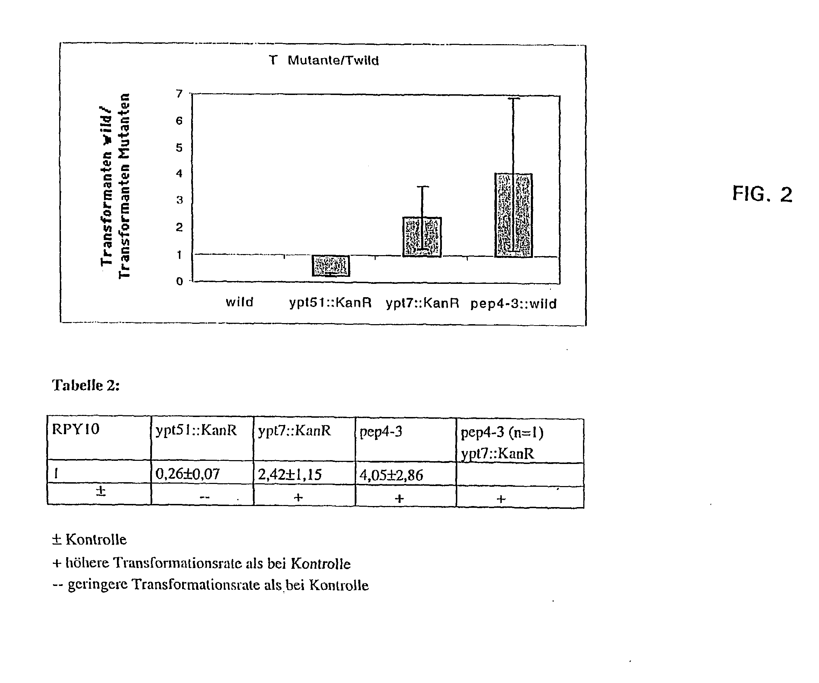

Eine Untersuchung mit mutanten Stämmen wurde wie folgt durchgeführt. 1*10⁹ Zellen aus der logarithmischen Wachstumsphase des Stammes RPY10 (Matα, leu2-3, ura3, his4, ade6) (ca. 5*10⁷ Zellen pro ml) sowie Deletionsmutanten des Stammes mit Deletion der Gene:
YPT51 (Singer-Krüger B., Stenmark H., Düsterhöft A.,
Phillipsen P., Yoo J.-S., Gallwitz D., Zerial M. (1994) Role of three rab5-like GTPases, ypt51p, yptslp, and ypt53p, in the endocytic and vacuolar protein sorting pathway of yeast. JCB, 125 (2) :283-298)
YPT7 (Wichmann H. et al, Hengst L., Gallwitz D. (1992) Endocytosis in yeast. evidence for the involvement of a small GTP-binding protein (Ypt7p). Cell 71(7):1131-42
VPS27 (Piper R.C., Cooper A.A., Yang H., Stevens T.H. (1995) Vps27 controls vacoular and endocytic traffic through a prevacuolar compartment *in Saccharomyces cerevisiae.* JCB, 131 (3) : 603-617
PEP4 (Meussdoerffer F., Tortora P., Holzer H. (1980) Purification and properties of proteinase A from yeast. J. Biol. Chem, 255 (24): 12087-93) In diesem Fall der Stamm SF838-9D, die pep4-3-Mutante des Stammes RPY10 (Piper et al. (1995)) SF838-9D mit einer ypt7-Deletion oder andere Kombinationen zweier Defekte in einem Stamm
wurden wie in Beispiel 1 behandelt. Zur Auswertung wurden sämtliche Transformationsraten auf den Stamm RPY10, der keinen Defekt in den Genen des Endocytoseweges hat, normiert. Die Ergebnisse (n=3) sind in der Abbildung 2 sowie der Tabelle 2 angegeben.

Insgesamt wird der Transportfluß von endocytotisch aufgenommenen Stoffen in Hefe durch die Mutanten wie folgt beeinflußt, wobei in dem zugrundeliegenden vereinfachten Modell für die Transportschritte für die Flüssigphasenendocytose und die Rezeptorvermittelte Endocytose weitgehend Übereinstimmung angenommmen wird:

Im Wildtyp sammeln sich die zu endocytierenden Makromoleküle zuerst an der Zellmembran, binden gegebenfalls an spezifische Rezeptoren, häufen sich an bestimmten Bereichen an. Es folgt die Internalisierung, d.h. die Aufnahme in die Zelle über Plasmamembraneinstülpungen, wobei auch extrazelluläre Flüssigkeit und darin gelöste Stoffe aufgenommen werden. Nach erfolgreicher Internalisierung tauchen die endocytierten Moleküle in den peripheren Endosomen auf, die unmittelbar unterhalb der Plasmamebran liegen. Von da gelangen sie in die Perinuklear-Endosomen, die nahe am Kern liegen. Der Innenraum des Endosomenkompartiments ist leicht sauer (ATP-getriebene Protonenpumpe), wobei im allgemeinen gilt, daß interne Endosomen saurer sind als periphere. Von den Endosomen gelangen endocytierte Moleküle in ein prä-vakuolären Kompartiment, das auch mit Transportvesikeln aus dem Golgi-Apparat, die die für die Vakuolen bestimmten neusynthetisierten Proteine transportieren, gespeist wird. Vom prä-vakuolären Kompartiment, gelangen die endocytierten Komplexe in die Vakoule, wo sie degradiert werden.

Die Internalization kann durch die temperatursensitiven Mutanten end 3 und end 4 bei der permissiven Temperatur blockiert werden. Diese Blockade wird in der Literatur sowohl für den alpha-Faktor /alpha-Faktor-Rezeptor -Komplex beschrieben, als auch für den Flüssigphasen-Endocytose-Marker Lucifer Yellow. Die Blockade dieser Schrittes kann eine Aufnahme von Nukleinsäuren über Endocytose verhindern und als Kontrolle für den Mechanismus der Aufnahme dienen.

Der Transport zwischen den Endosomen und der Vakuole wird durch die Deletion der Gene YPT51, YPT7 und VPS27 blockiert. Dies führt zu einem Transportstau an ausgewählten Schlüsselpositionen des Endocytoseweges. Die Hefeproteine ypt51p und ypt7p zeigen eine hohe Homologie zu den Säugetierproteinen rab5 und rab7, membranassoziierte GTPasen, die Schlüsselrollen in den Endocytoseweg von Säugetierzellen haben. rab5 hat einen regulatorischen Effekt in den frühen Schritten des Transports zwischen der Plasmamembran und den Endosomen und rab7 in den späten Schritten des Endocytoseweges (Chavrier P., Parton R.G., Hauri H.-P., Simons K.; Zerial M. (1990) Localization of low molecular weight GTP binding proteins to exocytic and endocytic compartments. Cell 62: 317-329, Bucci). Für Hefezellen, die das Protein ypt51p nicht exprimieren, wird zusätzlich zu den bei Säugerzellen beschriebene Effekten eine verringerte Ansäuerung der Vakoule beschrieben. Bei dem YPT7-Deletionsstamm wird außerdem eine verzögerte Reifung der löslichen vakuolären Enzyme beschrieben. Die Deletion des Gen VPS27 führt zu einer Blockade des Endocytoseweges hinter dem prä-vakuolären Kompartment, was dort zu einer starken Anhäufung von Golgi-Proteinen und endocytierten Material in diesem Kompartment führt. Von der Proteinase A, die von dem Gen pep4 kodiert wird, und in der Vakuole lokalisiert ist, werden bestimmte Degradationsenzyme bei ihrer Ankunft in der Vakoule so proteolysiert, daß sie dann erst ihre Aktivität entfalten. Das Fehlen der Proteinase A führt also zu einer verringerten Hydrolyse in der Vakuole.

### Beispiel 3:

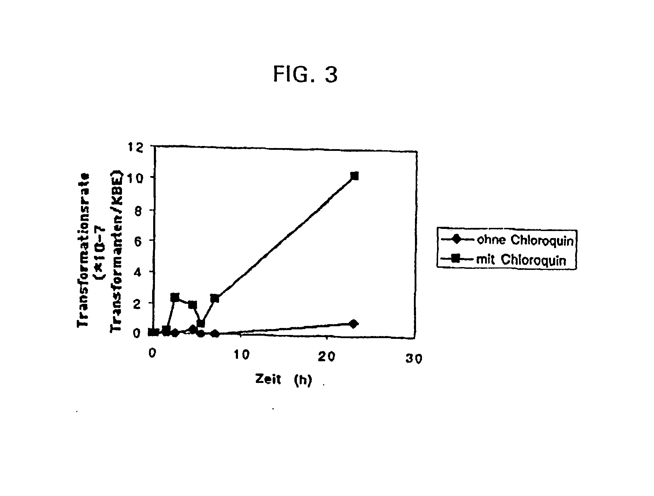

Es wurde wie in Beispiel 1 verfahren, jedoch in mehreren Versuchen die Inkubationszeit zwischen 0,5 und 24 h variiert. Die Ergebnisse der gemessenen Transformationsraten, mit und ohne Chloroquin, in Abhängigkeit von der Inkubationszeit sind in der Fig. 3 dargestellt.

### Beispiel 4:

1*10⁹ Zellen aus der logarithmischen Wachstumsphase des Stammes RH144-3D (Matα, leu2, ura3, his4) (4*10⁷-7*10⁷ Zellen pro ml)(Raths, S., Rohrer, J., Crausaz, F., Riezmann, H., 1993, JBC, vol. 120(1), 55-65) wurden mit 10 µg des gereinigten Plasmids YEP24 (siehe: D. Botstein et al., 1979, Gene 8:17-24) etwa 4h inkubiert.

Die Zellen wurden vor der Inkubation mit dem Plasmid mit mindestens 2x dem gleichem Volumen H₂O gewaschen und in 1 ml 1 M Saccharose (pH 3,3, hohe Konzentrationen fermentierbarer Zucker) aufgenommen, mit der Pipettenspitze resuspendiert und in je ein 10 ml Falkonröhrchen überführt. Sofort nach diesem Schritt wurden in alle Röhrchen 5 µl Chloroquin als Hilfsstoff (Stammlsg. 100 mM) dazupipettiert. Alle Röhrchen wurden 5 min bei Raumtemperatur inkubiert.

Nach Inkubation wurden alpha Faktor in einer Endkonzentration von 1 µM und 10 µg DNA und als Kontrolle in ein zweites Röhrchen nur 10 µg DNA aus derselben DNA-Aufbereitung dazupipettiert (in 3-100 µl). Die Falkon-Röhrchen wurden fest zugeschraubt und mindestens 2,5 Stunden in Wasserbad oder Brutraum schütteln gelassen bei 24°C.

Nach der Inkubation wurden 200-500 Zellen auf YE-Platten (Vollmedium) pro Platte ausplattiert und die koloniebildenden Einheiten (KBE)bestimmt. Die restlichen Zellen wurden auf 3-5 Platten mit Selektionsmedium ausplattiert. Nach 5 Tagen bei 24°C wurden die KBE ausgezählt und mittels PCR geprüft.

Dabei ergab sich für 1 µM alpha Faktor Endkonzentration ein Wert von 1,56*10⁻⁷ Transformationen/KBE und ohne alpha Faktor ein Wert von 0,8*10⁻⁷ Transformationen/KBE als Transformationsrate.

Der alpha Faktor / alpha Faktor Rezeptor Komplex wird endocytotisch aufgenommen und in der Vakuole degradiert. Der alpha Faktor kann einerseits als Ligand so modifiziert werden, daß (Makro-) Moleküle daran gekoppelt werden können, ohne die Aufnahmerate zu vermindern oder diese sogar zu verstärken. Andererseits ist es möglich, die Aufnahmerate von nicht an den Liganden gekoppelter DNA durch Zugabe des Liganden und der damit einhergehenden Induktion des Endocytoseprozesses zu verstärken.

### Beispiel 5:

Die humane Zellinie HepG2 wurde unter Verwendung des kommerziell erhältlichen Transferrinfektions-Systems mittels gerichteter Endocytose nach Vorschrift mit einem Vektor transfiziert, welcher sowohl cDNA für EGFP als auch cDNA für die Firefly-Luciferase, jeweils unter der Kontrolle eines konstitutiv aktiven viralen Promotors, enthält.

Die transfizierten Zellen wurden 48 h nach der Transfektion 3x mit PBS gewaschen, für 2 min. mit 0,25% Trypsin in PBS inkubiert, von der Zellkulturschale abgelöst und mit frischem Medium mit 10% FKS (fötales Kälberserum) versehen. Aliquots der Zellen wurden wie folgt untersucht:
a) Die Menge der gesamten DNA (oberflächengebunden und intrazellulär) wurde durch DNA Extraktion aus 10⁵ Zellen und anschließende quantitative PCR unter Verwendung einer definierten Menge des gleichen, gereinigten Plasmids als Referenzprobe bestimmt.
b) Weitere 10⁵ Zellen wurden für 30 min. bei 37°C mit 1 U/µl DNase I in 50 µl TBS/Mg (25 mM Tris-HCl, pH 7,5, 137 mM NaCl, 2,7 mM KCl, 10 mM MgCl₂) behandelt, in PBS gewaschen und anschließend wie in a) untersucht.
c) Weitere 10⁵ Zellen wurden wie in b) beschrieben mit DNase I behandelt, aber nach dem letzten Waschen nicht einer DNA Isolierung zugeführt, sondern ihre Zellkerne wurden zunächst wie in der Literaturstelle Dignam et al., Methods Enzymol., 101:582-598, 1983, beschrieben isoliert, um anschließend aus ihnen DNA zu gewinnen und diese quantitativ, wie unter a) beschrieben, zu bestimmen.
In a) bis c) diente ein zusätzlich durchgeführter quantitativer Nachweis zellulärer DNA, beispielsweise mittels quantitativer PCR, zur Standardisierung der Menge an aufgenommener DNA.
d) Knapp 48 h nach der Transfektion und kurz vor dem Waschen und Trypsinieren wurden die Zellen unter dem Fluoreszenzmikroskop auf den relativen Anteil grün fluoreszierender Zellen und damit auf die qualitative Transfektionseffizienz (Transfektionsrate) hin untersucht.
e) Ein weiteres Aliquot der Zellen wurde nach Herstellerangaben einem quantitativen Luciferase-Assay zugeführt.

Alle Untersuchungen a) bis e) wurden parallel an Zellen durchgeführt, die mit oder ohne Zugabe von Chloroquin transfiziert wurden. Auf diese Weise läßt sich unterscheiden, ob Chloroquin einen Einfluß auf die Gesamtmenge an adsorbierter und aufgenommer DNA (a), auf die Menge an intrazellulärer DNA (b), auf die Menge an kernständiger DNA (c), auf die Transfektionsrate (d) oder auf die Menge an Genprodukt (e) hat. Durch einen Vergleich der Ergebnisse läßt sich ermitteln, auf welcher Ebene Chloroquin die Transfektionseffizienz erhöht.

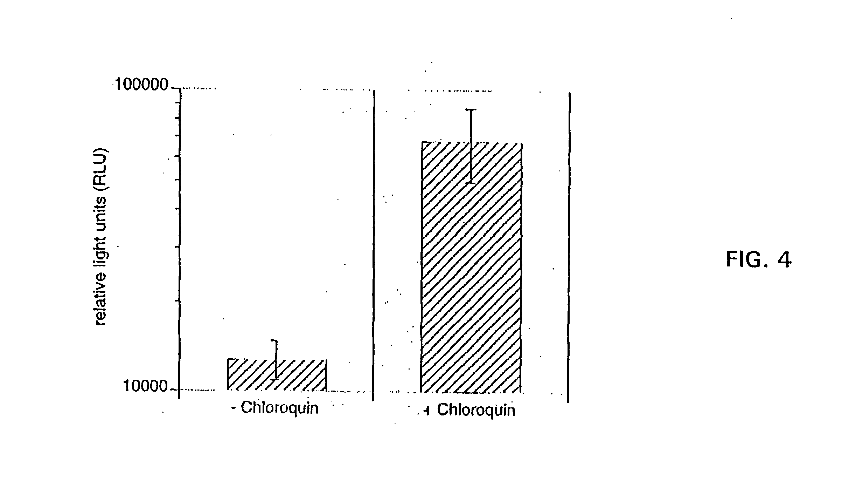

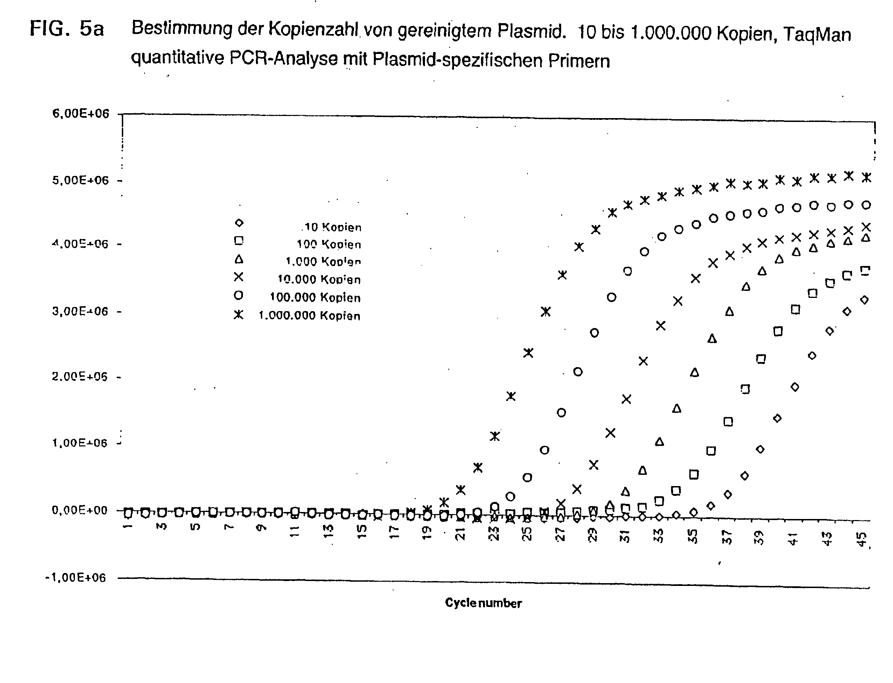

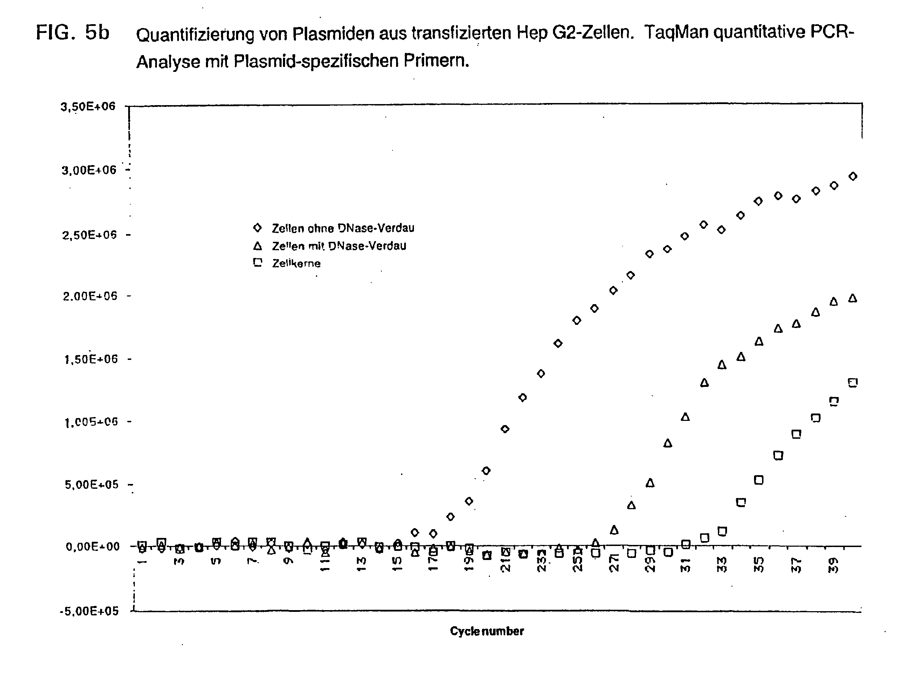

Die Ergebnisse sind in den Figuren 4 und 5a,b zusammengefaßt. In der Figur 4 ist der Einfluß von Chloroquin auf die Menge an exprimierbarer zellulär aufgenommener Vektor-DNA durch Messung der Luciferase Aktivität dargestellt. Mit Chloroquin wird eine fast 10-fache Aktivität gemessen. In der Figur 5 ist die Bestimmung der DNA-Menge in verschiedenen zellulären Kompartimenten mittels quantitativer PCR zusammengefaßt. Figur 5a ist eine Eichkurve. In der Figur 5b erkennt man die Mengen an DNA in verschiedenen Bereichen der Zellen. Die Rauten zeigen Werte aus der Stufe a), die Dreiecke aus der Stufe b) und die Quadrate aus Stufe c).

## Patentansprüche

1. Screeningverfahren zur Findung von die Endocytose von nieder- und/oder makromolekularen Verbindungen, insbesondere Nukleinsäuren, in Zellen fördernden Hilfsstoffen mit den folgenden Verfahrensschritten:
a) es wird ein Hefestamm ausgewählt und hieraus werden Hefezellen kultiviert,
b) die Hefezellen werden mit einem potentiellen, die Endocytose von nieder- und/oder makromolekularen Verbindungen, insbesondere Nukleinsäuren, in Zellen fördernden Hilfsstoff sowie mit einer replizierbaren und/oder im Kern und/oder im Cytosol der Hefezellen nachweisbaren Nukleinsäure oder nieder- und/oder makromolekularen Verbindungen inkubiert,
c) es wird die Transformationsrate oder die Transportrate in das Cytosol oder den Kern bestimmt und auf Erhöhung der Transformationsrate oder der Transportrate, verglichen mit der Transformationsrate oder der Transportrate in einem Referenzversuch ohne Einsatz des Hilfsstoffes, untersucht,
d) der Hilfsstoff wird selektiert oder verworfen nach Maßgabe des erhaltenen Wertes der Erhöhung der Transformationsrate oder der Transportrate in Stufe c).

2. Screeningverfahren nach Anspruch 1, wobei der Referenzversuch durchgeführt wird durch Ersatz der Verfahrenstufe b) durch die folgende Verfahrenstufe
b') die Hefezellen werden mit einer replizierbaren und/oder im Kern und/oder im Cytosol der Hefezellen nachweisbaren nieder- und/oder makromolekularen Verbindung oder Nukleinsäure inkubiert,
bei ansonsten unveränderten anderen Verfahrenstufen.

3. Screeningverfahren nach Anspruch 1 oder 2, wobei die folgende Verfahrenstufe angeschlossen wird:
e) ein in Stufe d) selektierter potentieller Hilfsstoff wird einer Bestimmung der Erhöhung der Transformationsrate oder der Transportrate gegenüber einer Transformationsrate oder Transportrate gegenüber Einsatz ohne Hilfsstoff bei Vertebratenzellen unterworfen und der Hilfsstoff wird selektiert oder verworfen nach Maßgabe der Erhöhung der Transformationsrate oder Transportrate.

4. Screeningverfahren nach einem der Ansprüche 1 bis 3, wobei eine Mehrzahl von unterschiedlichen potentiellen, die Endocytose von nieder- und/oder makromolekularen Verbindungen, insbesondere Nukleinsäuren, in Zellen fördernden Hilfstoffen parallel auf Erhöhung der Transformationsrate oder Transportrate untersucht wird.

5. Screeningverfahren nach einem der Ansprüche 1 bis 4, wobei parallel oder nacheinander die Erhöhung der Transformationsrate oder Transportrate für ein und denselben potentiellen Hilfsstoff mit wildtyp sowie mutierten Hefezellen durchgeführt wird.

6. Screeningverfahren nach einem der Ansprüche 1 bis 5, wobei der Hefestamm ausgewählt ist aus der Gruppe bestehend aus "RPY10, 4STLU, AH22, DBY747, GRF18, CBS6503, ABYS86, MF9, YMTA, BJ3505, GAG2", insbesondere RPY10 ist.

7. Screeningverfahren nach Anspruch 5 oder 6, wobei der mutierte Hefestamm ausgewählt ist aus der Gruppe bestehend aus "Deletionsmutanten des Stamms RPY10 mit Deletion der Gene YPT51, YPT7, VPS27 und/oder PEP4".

8. Screeningverfahren nach einem der Ansprüche 1 bis 7, wobei die Vertebratenzellinie ausgewählt ist aus der Gruppe bestehend aus "HepG2, K562, B16, MeWo, CMT-93, RMA, LL/2, 293, HT-1080, COS-1, COS-2, CV-1, CHO, OCM, HL-60, L929, 3T3, BLK CL.4, HeLa, T84", insbesondere HepG2 ist.

9. Screeningverfahren nach einem der Ansprüche 1 bis 8, wobei die Nukleinsäure ein Markergen enthält, vorzugsweise das Luciferasegen und/oder EGFP, und wobei die Erhöhung der Transformationsrate durch Auszählen der das Markergen exprimierenden Zellen bestimmt wird.

10. Screeningverfahren nach einem der Ansprüche 1 bis 8, wobei die Transformationsrate oder Transportrate auf die Anzahl teilungsfähiger Zellen aus der Stufe b) bezogen wird durch Bestimmung der Anzahl der kolonienbildenden Einheiten nach Kultivierung der Zellen aus Stufe b) in auf die Nukleinsäure abgestimmtem Selektionsmedium und Division durch die Anzahl der kolonienbildenden Einheiten nach Kultivierung der Zellen aus Stufe b) in Vollmedium.

11. Screeningverfahren nach einem der Ansprüche 1 bis 10, wobei eine Bestimmung von in das Cytosol oder in den Kern transportierten Nukleinsäuren mittels PCR durchgeführt wird.

12. Screeningverfahren zur Findung von die Endocytose von Nukleinsäuren in Zellen fördernden Hilfstoffen mit den folgenden Verfahrensschritten:
a) es wird ein Vertebratenzellenstamm ausgewählt und hieraus werden Zellen kultiviert,
b) die Zellen werden mit einem potentiellen, die Endocytose von Nukleinsäuren in Zellen fördernden Hilfsstoff sowie mit einer in den Vertebratenzellen transkribierbaren DNA und/oder mit einer direkt oder indirekt im Cytosol nachweisbaren Nukleinsäure inkubiert,
c) die Zellen der Stufe b) werden untersucht auf die Gesamtmenge aufgenommener und adsorbierter Nukleinsäure, die Menge an intrazellulärer Nukleinsäure, die Menge an kernständiger Nukleinsäure, die Transfektionsrate und/oder die Menge an Genprodukt, verglichen mit einem Referenzversuch ohne Einsatz des Hilfsstoffes,
d) der Hilfsstoff wird selektiert oder verworfen nach Maßgabe der Ergebnisse aus Stufe c).

## Claims

1. A screening method for finding auxiliary substances promoting the endocytosis of low and/or macro-molecular compounds, in particular nucleic acids, in cells, comprising the following steps:
a) a yeast strain is selected and yeast cells are cultivated therefrom,
b) the yeast cells are incubated with a potential auxiliary substance promoting the endocytosis of low and/or macro-molecular compounds, in particular nucleic acids, in cells, and with a nucleic acid being replicable and/or detectable in the nucleus and/or in the cytosol of the yeast cells or with low and/or macro-molecular compounds,
c) the transformation rate or the transportation rate into the cytosol or the nucleus is determined and examined for the increase of the transformation rate or the transportation rate, compared to the transformation rate or the transportation rate in a reference test without using the auxiliary substance,
d) the auxiliary substance is selected or rejected according to the obtained value of the increase of the transformation rate or the transportation rate in step c).

2. A screening method according to claim 1, wherein the reference test is performed by replacing the step b) by the following step:
b') the yeast cells are incubated with a low and/or macro-molecular compound or nucleic acid being replicable and/or detectable in the nucleus and/or in the cytosol of the yeast cells,
with otherwise unchanged other steps.

3. A screening method according to claim 1 or 2, wherein the following step is added:
e) a potential auxiliary substance selected in step d) is subjected to a determination of the increase of the transformation rate or the transportation rate compared to a transformation rate or transportation rate without using the auxiliary substance at vertebrate cells, and the auxiliary substance is selected or rejected according to the increase of the transformation rate or the transportation rate.

4. A screening method according to one of claims 1 to 3, wherein a multitude of different potential auxiliary substances promoting the endocytosis of low and/or macro-molecular compounds, in particular nucleic acids, in cells, are examined in parallel for the increase of the transformation rate or the transportation rate.

5. A screening method according to one of claims 1 to 4, wherein in parallel or one after the other the increase of the transformation rate or the transportation rate for one and the same potential auxiliary substance is performed with wildtype and mutated yeast cells.

6. A screening method according to one of claims 1 to 5, wherein the yeast strain is selected from the group consisting of "RPY10, 4STLU, AH22, DBY747, GRF18, CBS6503, ABYS86, MF9, YMTA, BJ3505, GAG2", in particular RPY10.

7. A screening method according to claim 5 or 6, wherein the mutated yeast strain is selected from the group consisting of "deletion mutants of the RPY10 strain with deletion of the genes YPT51, YPT7, VPS27 and/or PEP4".

8. A screening method according to one of claims 1 to 7, wherein the vertebrate cell line is selected from the group consisting of "HepG2, K562, B16, MeWo, CMT-93, RMA, LL/2, 293, HT-1080, COS-1, COS-2, CV-1, CHO, OCM, HL-60, L929, 3T3, BLK CL.4, HeLa, T84", is in particular HepG2.

9. A screening method according to one of claims 1 to 8, wherein the nucleic acid contains a marker gene, preferably a luciferase gene and/or EGFP, and wherein the increase of the transformation rate is determined by counting the cells expressing the marker gene.

10. A screening method according to one of claims 1 to 8, wherein the transformation rate or the transportation rate is related to the number of dividable cells from the step b) by determining the number of colony-forming units after the cultivation of the cells from the step b) in a selection medium matched with the nucleic acid and the division by the number of the colony-forming units after the cultivation of the cells from the step b) in full medium.

11. A screening method according to one of claims 1 to 10, wherein a determination of the nucleic acids transported into the cytosol or into the nucleus is performed by means of PCR.

12. A screening method for finding auxiliary substances promoting the endocytosis of nucleic acids in cells, comprising the following steps:
a) a vertebrate cell strain is selected and cells are cultivated therefrom,
b) the cells are incubated with a potential auxiliary substance promoting the endocytosis of nucleic acids in cells, and with a DNA transcriptable in the vertebrate cells and/or with a nucleic acid being directly or indirectly detectable in the cytosol,
c) the cells of the step b) are examined for the total amount of nucleic acid received and adsorbed, the amount of intracellular nucleic acid, the amount of nucleic acid in the nucleus, the transfection rate and/or the amount of gene product, compared to a reference test without using the auxiliary substance,
d) the auxiliary substance is selected or rejected according to the results from step c).

## Revendications

1. Procédé de screening pour trouver des substances auxiliaires promouvant l'endocytose de composés à bas poids moléculaire et/ou macromoléculaires, en particulier des acides nucléiques, dans les cellules, comprenant les étapes suivantes:
a) une souche de levure est choisie, et à partir de cela, des cellules de levure sont cultivées,
b) les cellules de levure sont incubées avec une substance auxiliaire potentielle promouvant l'endocytose de composés à bas poids moléculaire et/ou macromoléculaires, en particulier des acides nucléiques, dans les cellules, et avec un acide nucléique réplicable et/ou détectable dans le noyau et/ou dans le cytosol des cellules de levure ou des composés à bas poids moléculaire et/ou macromoléculaires,
c) le taux de transformation ou de transport dans le cytosol ou le noyau est déterminé et examiné pour savoir l'augmentation du taux de transformation ou de transport en comparaison avec le taux de transformation ou de transport dans un essai de référence sans utiliser la substance auxiliaire,
d) la substance auxiliaire est sélectionnée ou rejetée selon la valeur obtenue de l'augmentation du taux de transformation ou de transport dans l'étape c).

2. Procédé de screening selon la revendication 1, dans lequel l'essai de référence est effectué en remplaçant l'étape b) par l'étape suivante:
b') les cellules de levure sont incubées avec un composé à bas poids moléculaire et/ou macromoléculaire ou un acide nucléique réplicable et/ou détectable dans le noyau et/ou dans le cytosol des cellules de levure,
et à part cela, avec des étapes inchangées.

3. Procédé de screening selon la revendication 1 ou 2, dans lequel l'étape suivante est ajoutée:
e) une substance auxiliaire potentielle sélectionnée dans l'étape d) est soumise à une détermination de l'augmentation du taux de transformation ou de transport par rapport à un taux de transformation ou de transport sans utiliser la substance auxiliaire pour des cellules vertébrées, et la substance auxiliaire est sélectionnée ou rejetée selon l'augmentation du taux de transformation ou de transport.

4. Procédé de screening selon une des revendications 1 à 3, dans lequel une pluralité de différentes substances auxiliaires potentielles promouvant l'endocytose de composés à bas poids moléculaire et/ou macromoléculaires, en particulier des acides nucléiques, dans les cellules, est examinée en parallèle pour savoir l'augmentation du taux de transformation ou de transport.

5. Procédé de screening selon une des revendications 1 à 4, dans lequel en parallèle ou l'une après l'autre, l'augmentation du taux de transformation ou de transport est effectuée pour une seule et même substance auxiliaire potentielle avec des cellules de levure sauvages et mutées.

6. Procédé de screening selon une des revendications 1 à 5, dans lequel la souche de levure est sélectionnée à partir du groupe comprenant "RPY10, 4STLU, AH22, DBY747, GRF18, CBS6503, ABYS86, MF9, YMTA, BJ3505, GAG2", en particulier RPY10.

7. Procédé de screening selon la revendication 5 ou 6, dans lequel la souche de levure mutée est sélectionnée à partir du groupe comprenant "mutants de délétion de la souche RPY10 avec délétion des gènes YPT51, YPT7, VPS27 et/ou PEP4".

8. Procédé de screening selon une des revendications 1 à 7, dans lequel la ligne des cellules vertébrées est sélectionnée à partir du groupe comprenant "HepG2, K562, B16, MeWo, CMT-93, RMA, LL/2, 293, HT-1080, COS-1, COS-2, CV-1, CHO, OCM, HL-60, L929, 3T3, BLK CL.4, HeLa, T84", est en particulier HepG2.

9. Procédé de screening selon une des revendications 1 à 8, dans lequel l'acide nucléique comprend un gène marqueur, de préférence le gène luciférase et/ou EGFP, et dans lequel l'augmentation du taux de transformation est déterminée par comptage des cellules exprimant le gène marqueur.

10. Procédé de screening selon une des revendications 1 à 8, dans lequel le taux de transformation ou de transport est mis en relation au nombre des cellules divisibles de l'étape b) par détermination du nombre des unités formant colonies après la cultivation des cellules de l'étape b) dans un milieu de sélection s'accordant à l'acide nucléique et division par le nombre des unités formant colonies après la cultivation des cellules de l'étape b) dans un plein milieu.

11. Procédé de screening selon une des revendications 1 à 10, dans lequel une détermination d'acides nucléiques transportés dans le cytosol ou dans le noyau se fait au moyen de PCR.

12. Procédé de screening pour trouver des substances auxiliaires promouvant l'endocytose d'acides nucléiques dans les cellules, comprenant les étapes suivantes:
a) une souche de cellules vertébrées est choisie, et à partir de cela, des cellules sont cultivées,
b) les cellules sont incubées avec une substance auxiliaire potentielle promouvant l'endocytose d'acides nucléiques dans les cellules, et avec un ADN transcriptible dans les cellules vertébrées et/ou avec un acide nucléique détectable directement ou indirectement dans le cytosol,
c) les cellules de l'étape b) sont examinées pour savoir la quantité totale d'acide nucléique reçu et adsorbé, la quantité d'acide nucléique intracellulaire, la quantité d'acide nucléique dans le noyau, le taux de transfection et/ou la quantité de produit génique, en comparaison avec un essai de référence sans utiliser la substance auxiliaire,
d) la substance auxiliaire est sélectionnée ou rejetée selon le résultat de l'étape c).
